# EUROPEAN PATENT APPLICATION

(11) **EP 2 165 652 A1**
(43) Date of publication of application: **24.03.2010**
(21) Application number: 08772983.6
(22) Date of filing: 26.06.2008
(51) Int. Cl.: A61B 17/068, A61B 17/11, A61B 17/115

(54) **A SURGICAL PURSE-STRING STAPLE**

(30) Priority: 27.06.2007 CN 200720040100 U; 28.06.2007 CN 200720040150 U; 28.06.2007 CN 200720040149 U; 28.06.2007 CN 200720040148 U; 29.09.2007 CN 200720043964 U; 03.12.2007 CN 200720042033 U
(71) Applicant: Suzhou Touchstone International Medical Science Co., Ltd., Jiangsu 215021 (CN)
(72) Inventor: CHEN, Wangdong, Jiangsu 215021 (CN); SUN, Min, Jiangsu 215021 (CN); FAN, Xinyu, Jiangsu 215021 (CN); ZHOU, Jing, Jiangsu 215021 (CN); SUN, Kezhan, Jiangsu 215021 (CN)
(74) Representative: Gervasi, Gemma
(86) International application number: PCT/CN2008/001227
(87) International publication number: WO 2009/000161

(57) **Abstract**

A surgical purse-string suturing instrument comprises an upper jaw, jaw handle, grip, and a lower jaw, jaw handle, grip, wherein the jaws (1) and the corresponding jaw handles (3) are arranged in shape of "L" so that the doctor is able to clearly observe the placement of the tissues on the jaws (1) directly from one side of the jaw handle (3); in order to adapt to tissues of different widths, gaps (7), peaks (8) and valleys (9), or alternately arranged teeth (10) are disposed on the surfaces of the staple cartridges (2); by employing limiting rods (11) and anti-exceeding boss (13), the exceeding of tissues during the process of purse-string suturing can be effectively prevented so that the sutured purse-string has a complete shape.

## Description

### Technical Field of the Invention

The present invention relates to a surgical instrument used in surgery operations, more particularly, to a surgical purse-string suturing instrument, which belongs to the technical field of medical instrument.

### Background of the Invention

A purse-string suturing instrument is a medical instrument which is commonly used in surgery operations for intestines or other tissues, the performance of the purse-string suturing instrument directly affects the process of surgery operation, which is very important to the success of the surgery operation. In prior art, when doing suturing surgery operations with the stapler, an organ stump is normally purse-string sutured so that it can be securely ligated when the central rod of the stapler is placed, and the stapling operation can be started. There are two ways of operating purse-string suturing operations: one is manual suturing, of which the operation costs time and energy, stitches may go deeper or shallower and the stitch intervals are not consistent; the other is by purse-string suturing forceps, the needle leading the thread goes through the jaw which holds tissues so as to make purse-string suture, but it is not easy to operate when making the needle go through the small hole of the jaw, which is a disadvantage.

The automatic purse-string device is emerged which solves the above-mentioned problem to a large extent, however, defects are found during the operation process that need to be overcome. For example, with the purse-string device of prior art, jaw handles and jaws are connected to be a shape of "T", when the doctor observes whether the tissues are placed properly after the jaws are placed into the tissues, the sight is barriered by the jaw handles, it needs to left and right swing the jaw handles so that both sides of the jaws can be seen, which is not good for the successful implementation of the surgery operation; furthermore, the size of automatic purse-string device is limited, but the widths of tissues are various, for some special operation positions, the bigger sized device can not be put in while the smaller one may have some tissues left out off the device which affects the device being triggered; besides, the automatic purse-string device of prior art is lack of limitation to the exceeding tissues, thus the tissues may exceed, which is not good for shaping the purse-string.

### Summary of the Invention

The invention is aimed at solving the problems of prior art by providing a surgical purse-string suturing instrument.

The object of the present invention is achieved by the following technical scheme: a surgical purse-string suturing instrument comprises an upper jaw, jaw handle, grip, and a lower jaw, jaw handle, grip, wherein the upper jaw, the lower jaw handle and the lower grip are molded into one injection part, the lower jaw, the upper jaw handle and the upper grip are molded into another injection part, the two injection parts are hinged together in shape of a pair of scissors at the jaw handles, an upper staple cartridge and a lower staple cartridge are disposed respectively inside the upper jaw and the lower jaw, the upper staple cartridge and the lower staple cartridge form a suturing area, wherein the jaw handle is connected with a side end of the corresponding jaw in each of said two injection parts, the upper jaw and the upper jaw handle are arranged in shape of "L", the lower jaw and the lower jaw handle are arranged in shape of "L".

Preferably, in the surgical purse-string suturing instrument, the angle between the jaw handle and the axis of the corresponding jaw is from 90°to 180°.

Preferably, in the surgical purse-string suturing instrument, at least one gap is disposed respectively on the surfaces of the upper and lower staple cartridges, a corresponding gap is disposed respectively on the jaws at a proper location; alternatively, peaks and valleys are disposed on the surfaces of the upper and lower staple cartridges, the peaks engage with the valleys so that the suturing area formed between the upper staple cartridge and the lower staple cartridge is in shape of wave; alternatively, alternately arranged teeth with a height from 0.5mm to 2.0mm are disposed on the surfaces of the upper and lower staple cartridges.

Preferably, in the surgical purse-string suturing instrument, limiting devices are disposed on both of two ends of the jaws. All said limiting devices are limiting rods, of which one limiting rod is fixed with the upper jaw or the lower jaw and pivotally connected with the jaw; or one limiting rod is connected with the upper and lower jaws in a detachable way by means of two insert blocks; or one end is installed with the limiting rod, the other end is installed with an anti-exceeding boss and a move over groove, the anti-exceeding boss and the move over groove are disposed at corresponding locations on the two jaws and have shapes mating with each other. More preferably, the anti-exceeding boss is structured in shape of rectangle or trapezium.

The prominent and substantive distinguishing features and marked improvement of the present invention are as follows: because the jaws and the corresponding jaw handles are arranged in shape of "L", when implementing the surgery operation, the doctor is able to clearly observe the placement of tissues on the jaws directly from one side of the jaw handle, the sight will not be barriered by the jaw handles, there is no need to left and right swing the jaw handles to observe whether the tissues are properly clamped by the two sides of the jaws, which reduces the difficulties when the doctor implementing the surgery operation. Furthermore, gaps, peaks and valleys, or up and down staggeredly arranged teeth are disposed on the surface of the staple cartridge so as to adapt to tissues of different widths; the tissues can easily occupy the whole surface of the staple cartridge so that tissues purse-string sutured will not be left out. Besides, by disposing limiting rods and anti-exceeding boss, the exceeding of tissues during the process of purse-string suturing can be effectively prevented, especially for the surgery locations of comparatively smaller space but comparatively larger tissue width, finally the tissues can be purse-string sutured to have a complete shape.

### Brief Description of the Drawings

The objects, features and advantages of the present invention will become apparent from the following detailed description of the preferred embodiments, with references to the appended drawings, which are intended for illustrative but not limitative purposes. While the embodiments are provided as typical technical solutions of the present invention, various modifications, alternative constructions and equivalents may be employed without departing from the true spirit and scope of the present invention.
FIG. 1 is a schematic view showing the overall structure of the surgical purse-string suturing instrument according to the first embodiment of the present invention;
FIG. 2 is a structural view showing the jaw of the surgical purse-string suturing instrument according to the second embodiment of the present invention;
FIG. 3 is a structural view showing the staple cartridge of the surgical purse-string suturing instrument according to the second embodiment of the present invention;
FIG. 4 is a structural view showing the staple cartridge of the surgical purse-string suturing instrument according to the third embodiment of the present invention;
FIG. 5 is another structural view showing the staple cartridge of the surgical purse-string suturing instrument according to the third embodiment of the present invention;
FIG. 6 is a structural view showing the staple cartridge of the surgical purse-string suturing instrument according to the fourth embodiment of the present invention;
FIG. 7 is another structural view showing the staple cartridge of the surgical purse-string suturing instrument according to the fourth embodiment of the present invention;
FIG. 8 is a schematic view showing a partial structure of the surgical purse-string suturing instrument according to the fifth embodiment of the present invention;
FIG. 9 is a schematic view showing a partial structure of the surgical purse-string suturing instrument according to the sixth embodiment of the present invention;
FIG. 10 is another schematic view showing a partial structure of the surgical purse-string suturing instrument according to the sixth embodiment of the present invention;
FIG. 11 is a schematic view showing a partial structure of the surgical purse-string suturing instrument according to the seventh embodiment of the present invention.

In which:
- 1: jaw
- 3: jaw handle
- 5: catch
- 7: gap
- 9: valley
- 11: limiting rod
- 13: anti-exceeding boss

- 2: staple cartridge
- 4: grip
- 6: safety lever
- 8: peak
- 10: teeth
- 12: insert block
- 14: move over groove

### Detailed Description of the Preferred Embodiments

### First Embodiment

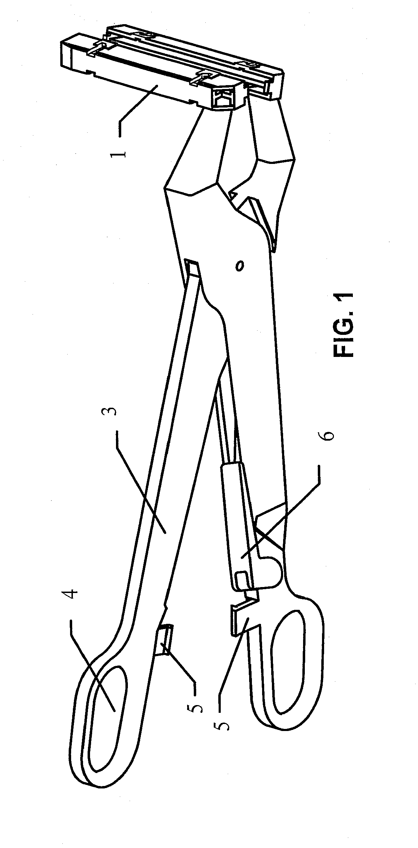

As illustrated in Figure 1, a surgical purse-string suturing instrument comprises an upper jaw, jaw handle, grip, and a lower jaw, jaw handle, grip,, wherein the upper jaw, the lower jaw handle and the lower grip are molded into one injection part, the lower jaw, the upper jaw handle and the upper grip are molded into another injection part, the two injection parts are hinged together in shape of a pair of scissors at the jaw handles, an upper staple cartridge and a lower staple cartridge are disposed respectively inside the upper jaw and the lower jaw (which are not shown in the figure), the upper staple cartridge and the lower staple cartridge form a suturing area, wherein the jaw handle 3 is connected with a side end of the corresponding jaw in each of said two injection parts, the upper jaw and the upper jaw handle are arranged in shape of "L", the lower jaw and the lower jaw handle are arranged in shape of "L", the angle between the jaw handle and the axis of the corresponding jaw is from 90 °to 180°. A pair of engageable catches 5 are disposed at the corresponding location of the upper and lower jaw handles 3 so that the jaws can tightly clamp the tissues during operation process and keep in a comparatively steady state. At the same time, a safety lever 6 is disposed on one of the jaw handle 3.

when assembling, the upper and lower jaws 1 are superimposed face to face at first, the two injection parts are hinged by the pin, then the safety lever 6 is disposed on one side of one jaw handle 3 and opened, the staple-pushing sheet and purse staple are disposed into the upper and lower staple cartridges, the purse string is disposed in the string-holding groove on the surfaces of the staple cartridges, at last the upper and lower staple cartridges are disposed in the upper and lower jaws 1. During the process of surgery operation, the doctor opens the purse-string suturing instrument by the upper and lower grips 4 so that the upper and lower jaws 1 are opened, then the doctor places the tissues for purse-string suturing in the center between the two upper and lower jaws 1 by going through the opening side, then the safety lever 6 is released, the upper and lower grips 4 are tightly held until the pair of catches 5 are engaged with each other so that the upper and lower jaws 1 are completely closed, at last the catches 4 and grips 4 are opened and the purse-string suturing instrument is moved out, the purse string is tightened up, thus the tissues are purse-string sutured.

### Second Embodiment

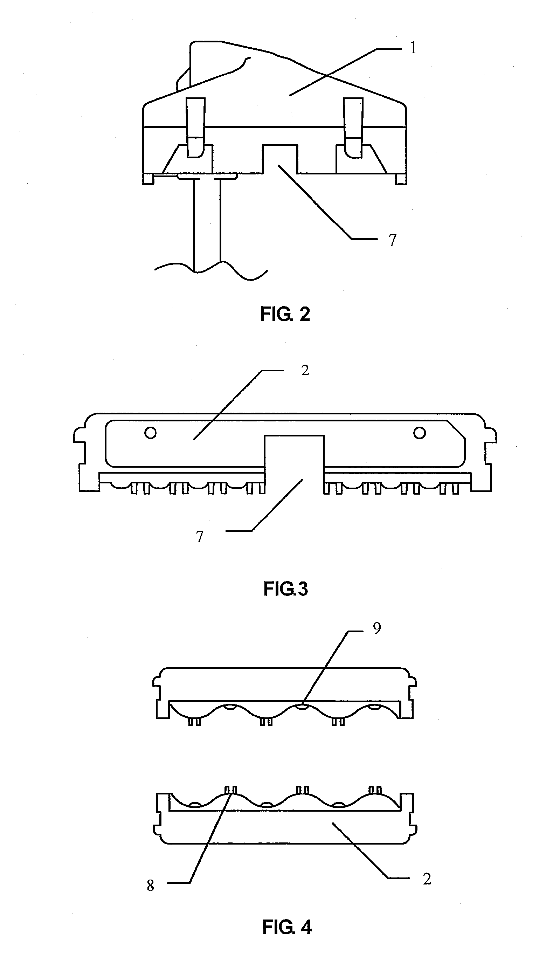

In the second embodiment of the present invention, a further preferred structure of the jaws and the staple cartridges is illustrated in figures 2 and 3, that is, at least one gap 7 is disposed respectively on the surfaces of the upper and lower staple cartridges 2, and one corresponding gap 7 is also disposed on the jaws 1 at the proper location on the staple cartridges 2, more specifically, the number of gaps 7 disposed on the upper staple cartridge 2 and the lower staple cartridge 2 is equal, the upper and lower gaps 7 are disposed at the corresponding positions and paired; alternatively, the staple cartridges 2 have one pair of gaps 7 which are disposed respectively at random locations of the upper and lower staple cartridges 2; alternatively, this pair of gaps 7 can be disposed in the middle, on the left or right of the upper and lower staple cartridges 2 according to requirements. There can be two pairs of gaps 7 which are disposed respectively on the left and right sides of the upper and lower staple cartridges 2 in order to handle some special tissue cuts.

By employing this structure, during the process of purse-string suturing, if the width of the cut tissues is larger than the width of the staple cartridges 2, the exceeding tissues can be placed in the gap 7 to make the tissues occupy the whole surface of the staple cartridges 2 so that all tissue cuts requiring purse-string suture are placed in the good positions for suturing, then the similar operation steps as the first embodiment are carried out to have the tissues purse-string sutured. Usually, one pair of upper and lower gaps 7 are disposed, also, the locations and numbers of gaps 7 can be changed so as to meet different requirements of purse-string suture on tissue cuts, thereby the optimal suturing effects are ensured and also the success implementation of the surgery operation is ensured.

### Third Embodiment

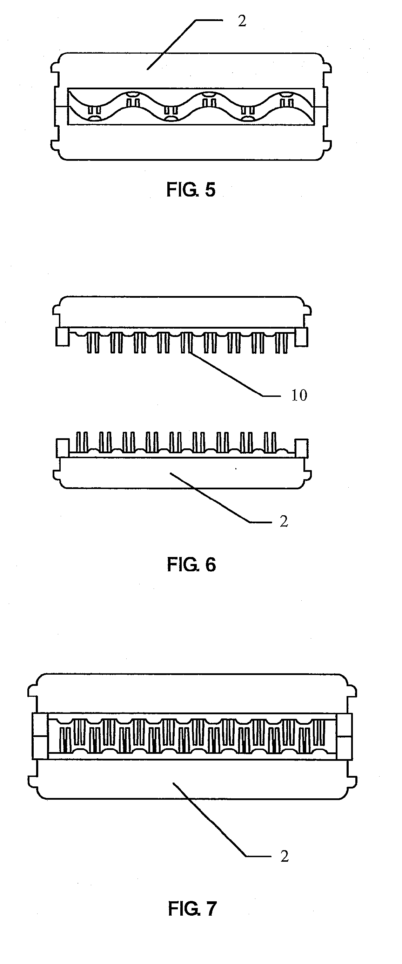

In the third embodiment of the present invention, a further preferred structure of the staple cartridges 2 is illustrated in figures 4 and 5, that is, the surfaces of staple cartridges 2 have peaks 8 and valleys 9, and the peaks 8 mate with the valleys 9 so that the suturing area formed between the upper staple cartridge and the lower staple cartridge is in shape of wave. Furthermore, at least one peak 8 and valley 9 are disposed between the upper staple cartridge and the lower staple cartridge; when a plurality of peaks 8 and valleys 9 are both disposed on the surface of the same one staple cartridge, it is more preferably that the peaks 8 and valleys 9 are arranged at same intervals. In order to adapt to special tissues, the relative heights (depths) between peaks 8 and valleys 9, peak and peak, valley and valley can be different so that the largest contact surfaces can be purse-string sutured. By employing this structure, the contact surfaces between the staple cartridges and the tissues are effectively increased, even though the width of tissues are slightly bigger than the width of the staple cartridges, the tissues will not exceed because the relative length of the staple cartridges is increased by employing the wave structure.

### Fourth Embodiment

In the fourth embodiment of the present invention, the staple cartridges 2 employ another structure as illustrated in figures 6 and 7, that is, teeth 10 are disposed on the suturing area of the staple cartridges 2, said teeth have a height from 0.5mm to 2.0mm, the teeth 10 of the upper and lower staple cartridges are arranged alternatively. By employing this structure, when the upper and lower staple cartridges engages with each other, the tissues clamped in the middle therein are in shape of wave so that the contact surface between the staple cartridges 2 and the tissues is increased, the problem of tissue exceeding caused by the width of tissues slightly bigger than the width of the staple cartridges 2 is effectively solved.

### Fifth Embodiment

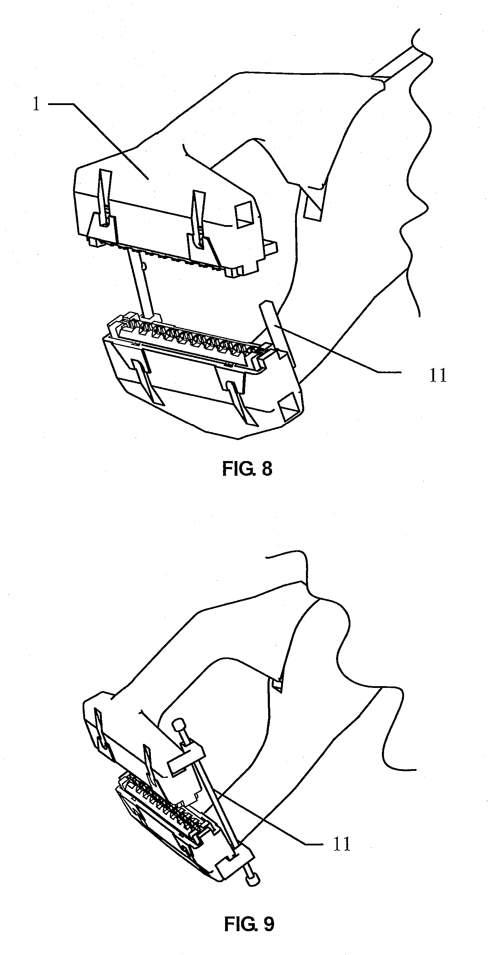

In the fifth embodiment of the present invention, limiting devices are disposed on both of two ends of the upper and lower jaws 1, for example: a limiting rod is disposed at one end for positioning of the upper and lower jaws 1 when assembling, another limiting rod 11 is disposed at the other end as illustrated in Figure 8. Both of these two limiting rods can be disposed on the inner sides of the two ends of the jaws 1, the limiting rod 11 can be fastened on the surface of one of the jaw and pivotally connected with the jaw so that the limiting rod 11 is able to rotate at a certain degree such as to rotate outward or rotate backward, a gap is formed after rotation which helps the doctor to place tissue cuts in the center of the suturing area between the upper and lower staple cartridges, the limiting rod 11 is reset after the tissues are places, then the purse-string suture is started. Because limiting devices are disposed on both ends of the jaws 1, the tissues will not exceed.

### Sixth Embodiment

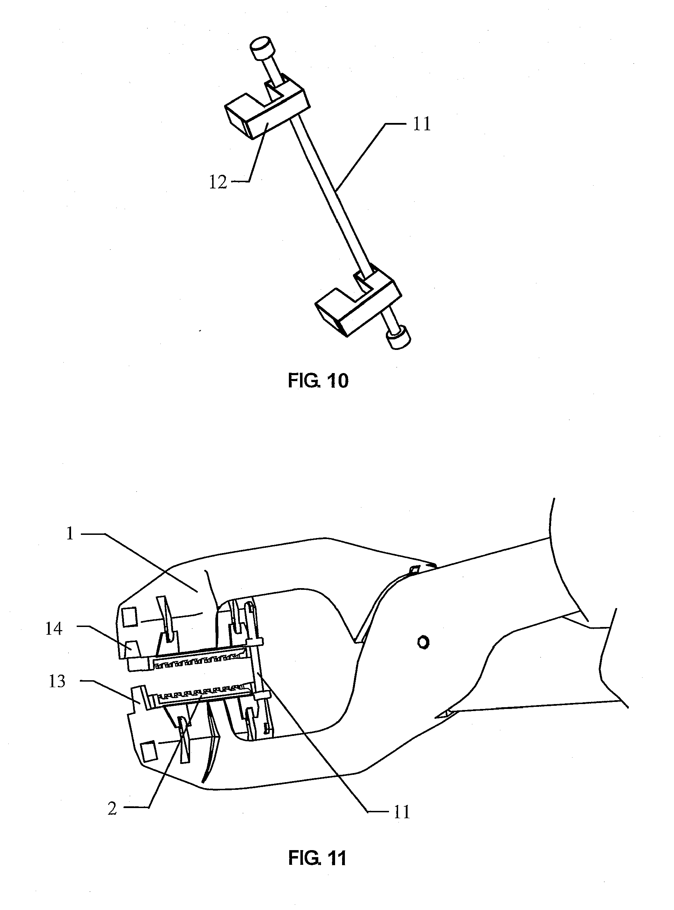

The sixth embodiment is similar to the fifth embodiment; limiting devices are disposed on both of two ends of the upper and lower jaws 1, the limiting rod 11 at one end is not fastened with the jaws 1 but connected with the jaws 1 in a detachable way by means of auxiliary parts as illustrated in figures 9 and 10, that is, the auxiliary parts are the upper and lower insert blocks 12, these two insert blocks 12 are respectively inserted on the side ends of the upper and lower jaws 1 at a certain inclined angle, the angle mates with the angle of the string-holding groove on the surfaces of the upper and lower staple cartridges; at the same time, positioning holes 12 are disposed on the insert blocks 12 and the limiting rods 11 go through the limiting holes. In order to prevent the limiting rods 11 from getting loose, diameters of the cross sections of the limiting rods 11 are larger than the diameters of the limiting holes. By employing the detachably connected structure, it is convenient for replacement and the limiting rods can be moved easily to other sized automatic purse-string devices for usage. During the process of operation, the tissues which are likely to exceed are limited by the limiting rods 11 within the suturing area between the upper and lower jaws 1, which is good for the success implementation of the surgery operation.

### Seventh Embodiment

In the seventh embodiment of the present invention, the anti-exceeding structure is different from that in the fifth embodiment and the sixth embodiment. As illustrated in Figure 11, a limiting rod 11 is disposed on one end of the upper and lower jaws 1, a boss 13 and a move over groove 14 are disposed on the other end, the width of the move over groove 14 is slightly larger than the width of the anti-exceeding boss 13, the positions of the boss 13 and the move over groove 14 correspond to each other, which can be interchanged up and down. According to specific conditions, the anti-exceeding boss 13 can be structured in shape of rectangle or trapezium so as to limit the exceeding of the tissues.

## Claims

1. A surgical purse-string suturing instrument, comprising an upper jaw (1), jaw handle (3), grip (4), and a lower jaw (1), jaw handle (3), grip (4), wherein the upper jaw (1), the lower jaw handle (3) and the lower grip (4) are molded into one injection part, the lower jaw (1), the upper jaw handle (3) and the upper grip (4) are molded into another injection part, the two injection parts are hinged together in shape of a pair of scissors at the jaw handles (3), an upper staple cartridge (2) and a lower staple cartridge (2) are disposed respectively inside the upper jaw (1) and the lower jaw (1), the upper staple cartridge (2) and the lower staple cartridge (2) form a suturing area, wherein the jaw handle (3) is connected with a side end of the corresponding jaw (1) in each of said two injection parts, the upper jaw (1) and the upper jaw handle (3) are arranged in shape of "L", the lower jaw (1) and the lower jaw handle (3) are arranged in shape of "L".

2. The surgical purse-string suturing instrument according to claim 1, wherein the angle between the jaw handle (3) and the axis of the corresponding jaw (1) is from 90° to 180°.

3. The surgical purse-string suturing instrument according to claim 1, wherein at least one gap (7) is disposed respectively on the surfaces of the upper and lower staple cartridges (2), a corresponding gap (7) is disposed respectively on the jaws (1) at a proper location.

4. The surgical purse-string suturing instrument according to claim 1, wherein peaks (8) and valleys (9) are disposed on the surfaces of the upper and lower staple cartridges (2), the peaks (8) engage with the valleys (9) so that the suturing area formed between the upper staple cartridge (2) and the lower staple cartridge (2) is in shape of wave.

5. The surgical purse-string suturing instrument according to claim 1, wherein alternately arranged teeth (10) with a height from 0.5mm to 2.0mm are disposed on the surfaces of the upper and lower staple cartridges (2).

6. The surgical purse-string suturing instrument according to claim 1, wherein limiting devices (11) are disposed on both of two ends of the upper and lower jaws (1).

7. The surgical purse-string suturing instrument according to claim 6, wherein all said limiting devices (11) are limiting rods, of which one limiting rod is fixed with the upper jaw (1) or the lower jaw (1) and pivotally connected with the jaw.

8. The surgical purse-string suturing instrument according to claim 6, wherein all said limiting devices (11) are limiting rods, of which one limiting rod is connected with the upper and lower jaws (1) in a detachable way by means of two insert blocks (12).

9. The surgical purse-string suturing instrument according to claim 6, wherein said limiting devices (11), one end is installed with the limiting rod, the other end is installed with an anti-exceeding boss (13) and a move over groove (14), the anti-exceeding boss (13) and the move over groove (14) are disposed at corresponding locations on the two jaws (1) and have shapes mating with each other.

10. The surgical purse-string suturing instrument according to claim 9, wherein said anti-exceeding boss (13) is structured in shape of rectangle or trapezium.
